# EUROPÄISCHE PATENTSCHRIFT

(11) **EP 0 531 821 B1**
(45) Veröffentlichungstag und Bekanntmachung des Hinweises auf die Patenterteilung: **31.01.1996**
(21) Anmeldenummer: 92114683.3
(22) Anmeldetag: 28.08.1992
(51) Int. Cl.: C07C 69/734, C07C 67/343, A01N 37/10

(54) **1-Alkoxyhexatrien-2-carbonsäureester**
1-Alkoxyhexatriene-2-carboxylic acid esters
Esters de l'acide 1-alkoxyhextriène-2-carboxylique

(30) Priorität: 10.09.1991 DE 4129962
(43) Veröffentlichungstag der Anmeldung: 17.03.1993
(73) Patentinhaber: BAYER AG, D-51368 Leverkusen (DE)
(72) Erfinder: Gayer, Herbert, Dr., W-4019 Monheim-Bamberg (DE); Gerdes, Peter, Dr., W-5100 Aachen (DE); Dehne, Heinz-Wilhelm, Dr., W-4019 Monheim (DE); Dutzmann, Stefan, Dr., W-4010 Hilden (DE)

(56) Entgegenhaltungen:
- EP-A- 0 421 102
- DE-A- 4 012 792
- TETRAHEDRON LETT. Bd. 30, Nr. 40, 1989, Seiten 5417 - 5420 M. SUTTER
- J. ANTIBIOT. Bd. 43, Nr. 6, 1990, Seiten 655 - 660 A. FREDENHAGEN ET AL

## Beschreibung

Die Erfindung betrifft neue 1-Alkoxyhexatrien-2-carbonsäureester, mehrere Verfahren zu ihrer Herstellung und ihre Verwendung als Schädlingsbekämpfungsmittel.

Es ist bekannt, daß bestimmte 1-Alkoxyhexatrien-2-carbonsäureester wie beispielsweise die Verbindung 1-Methoxy-3-methyl-6-phenyl-hexatrien-2-carbonsäuremethylester oder die Verbindung 1-Methoxy-3-methyl-6-(4-methoxyphenyl)-hexatrien-2-carbonsäuremethylester fungizide Eigenschaften besitzen (vergleiche z. B. Chem. Ber. 111, 2779-2784 [1978]; Liebigs Ann. Chem., 1984, 1616-25; DE 30 25 368; Collect. Czech. Chem. Commun., 47, 1020-1025 [1982]; Collect. Czech. Chem. Commun., 48, 1508-1512 [1983]).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue 1-Alkoxyhexatrien-2-carbonsäureester der allgemeinen Formel (I), 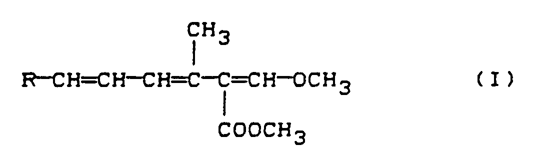
in welcher
- R: für substituiertes Phenyl steht, wobei als Substituerten infrage kommen:
Halogen, Cyano, Nitro, Alkyl, Alkenyl, Alkinyl, Alkoxy, Alkenyloxy, Alkinyloxy, Alkylthio, Alkylsulfinyl, Alkylsulfonyl, Halogenalkyl, Halogenalkenyl, Halogenalkinyl, Halogeralkoxy, Halogenalkenyloxy, Halogenalkinyloxy, Halogenalkylthio, Halogenalkylsulfinyl, Halogenalkylsulfonyl, Alkoxycarbonyl, Alkoxycarbonylalkyl, Alkoximinoalkyl sowie jeweils gegebenenfalls substituiertes Aralkyl, Aralkyloxy, Aryl oder Aryloxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind,
gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen 1-Alkoxyhexatrien-2-carbonsäureester der allgemeinen Formel (I), 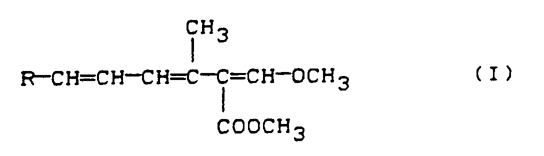
in welcher
- R: die oben angegebene Bedeutung hat
erhält, wenn man Methoxymethylencarbonsäureester der Formel (II), 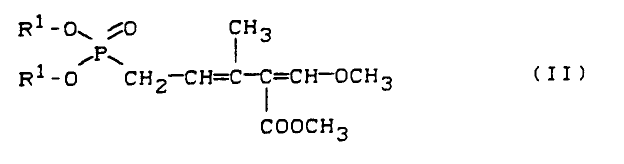
in welcher
- R¹: für Alkyl steht,
mit Benzaldehyden der Formel (III),

R-CH=O (III)

in welcher
- R: die oben angegebene Bedeutung hat,
gegebenenfalls in Gegenwart eines Verdünnungsmittel und gegebenenfalls in Gegenwart eines Reaktionshilfsmittels umsetzt.

Schließlich wurde gefunden, daß die erfindungsgemäßen 1-Alkoxyhexatrien-2-carbonsäureester der allgemeinen Formel (I) gute Wirksamkeit gegenüber pflanzenschädigenden Mikroorganismen besitzen.

Überraschenderweise zeigen die erfindungsgemäßen 1-Alkoxyhexatrien-2-carbonsäureester der allgemeinen Formel (I) eine erheblich bessere fungizide Wirksamkeit im Vergleich zu den aus dem Stand der Technik bekannten 1-Alkoxyhexatrien-2-carbonsäureestern, wie beispielsweise die Verbindung 1-Methoxy-3-methyl-6-phenyl-hexatrien-2-carbonsäuremethylester oder die Verbindung 1-Methoxy-3-methyl-6-(4-methoxyphenyl)-hexatrien-2-carbonsäuremethylester, welche chemisch und wirkungsmäßig naheliegende Verbindungen sind.

Die erfindungsgemäßen 1-Alkoxyhexatrien-2-carbonsäureester sind durch die Formel (I) allgemein definiert. Bevorzugt sind Verbindungen der Formel (I), bei welchen
- R: für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, jeweils geradkettiges oder verzweigtes Halogenalkyl, Halogenalkoxy oder Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 6 Kohlenstoffatomen und 1 bis 11 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder jeweils geradkettiges oder verzweigtes Alkyl und/oder Alkoxy mit jeweils 1 bis 6 Kohlenstoffatomen substituiertes Benzyl, Benzyloxy, Phenyl oder Phenoxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

Besonders bevorzugt sind Verbindungen der Formel (I), bei welchen
- R: für gegebenenfalls einfach bis fünffach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 4 Kohlenstoffatomen und 1 bis 7 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 4 Kohlenstoffatomen in den einzelnen Alkylteilen sowie gegebenenfalls einfach bis dreifach, gleich oder verschieden durch Halogen und/oder jeweils geradkettiges oder verzweigtes Alkyl und/oder Alkoxy mit jeweils 1 bis 4 Kohlenstoffatomen substituiertes Benzyl, Benzyloxy, Phenyl oder Phenyloxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

Ganz besonders bevorzugt sind Verbindungen der Formel (I), bei welchen
- R: für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, jeweils geradkettiges oder verzweigtes Alkenyl, Alkinyl, Alkenyloxy oder Alkinyloxy mit jeweils 2 bis 4 Kohlenstoffatomen, jeweils geradkettiges oder verzweigtes Halogenalkyl, Halogenalkoxy oder Halogenalkylthio mit jeweils 1 bis 3 Kohlenstoffatomen und 1 bis 7 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 4 Kohlenstoffatomen und 1 bis 5 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 3 Kohlenstoffatomen in den einzelnen Alkylteilen sowie gegebenenfalls einfach oder zweifach, gleich oder verschieden durch Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy substituiertes Benzyl, Benzyloxy, Phenyl oder Phenoxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

Verwendet man beispielsweise 5-(Diethoxyphosphinyl)-1-methoxy-3-methyl-1,3-pentadien-2-carbonsäuremethylester und 4-Chlorbenzaldehyd als Ausgangsstoffe, so läßt sich der Reaktionsablauf des erfindungsgemäßen Verfahrens durch das folgende Formelschema darstellen: 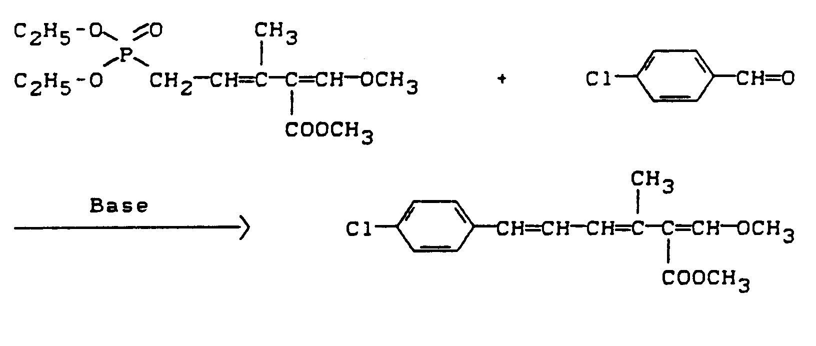
Die zur Durchführung des erfindungsgemäßen Verfahrens als Ausgangsstoffe benötigten Methoxymethylencarbonsäureester sind durch die Formel (II) allgemein definiert. In dieser Formel (II) steht R¹ vorzugsweise für geradkettiges oder verzweigtes Alkyl mit 1 bis 6, insbesondere 1 bis 3 Kohlenstoffatomen.
Die Methoxymethylencarbonsäureester der Formel (II) sind bekannt (vergleiche z. B. DE 40 12 792).

Die zur Durchführung des erfindungsgemäßen Verfahrens weiterhin als Ausgangsstoffe benötigten Benzaldehyde sind durch die Formel (III) allgemein definiert. In dieser Formel (III) steht R vorzugsweise für diejenigen Reste, die bereits im Zusammenhang mit der Beschreibung der erfindungsgemäßen Verbindungen der Formel (I) als bevorzugt für diesen Substituenten genannt wurden.

Die Benzaldehyde der Formel (III) sind allgemein bekannte Verbindungen der organischen Chemie.

Als Verdünnungsmittel zur Durchführung des erfindungsgemäßen Verfahrens kommen inerte organische Lösungsmittel infrage. Hierzu gehören insbesondere aliphatische, alicyclische oder aromatische Kohlenwasserstoffe, wie beispielsweise Benzin, Benzol, Toluol, Xylol, Petrolether, Hexan, Cyclohexan; Ether, wie Diethylether, Diisopropylether, Dioxan, Tetrahydrofuran oder Ethylenglykoldimethyl- oder -diethylether; Amide, wie N,N-Dimethylformamid, N,N-Dimethylacetamid, N-Methylformanilid, N-Methylpyrrolidon oder Hexamethylphosphorsäuretriamid oder Sulfoxid, wie Dimethylsulfoxid.

Das erfindungsgemäße Verfahren wird vorzugsweise in Gegenwart eines geeigneten Reaktionshilfsmittels durchgeführt. Als solche kommen alle üblichen anorganischen und organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-t-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat.

Die Reaktionstemperaturen können bei der Durchführung des erfindungsgemäßen Verfahrens (a) in einem größeren Bereich variiert werden. Im allgemeinen arbeitet man bei Temperaturen zwischen -70°C und 100°C, vorzugsweise bei Temperaturen zwischen -30°C und 50°C.

Zur Durchführung des erfindungsgemäßen Verfahrens setzt man pro Mol an Methoxymethylencarbonsäureester der Formel (II) im allgemeinen 1.0 bis 1.5 Mol, vorzugsweise 1.0 bis 1.2 Mol an Benzaldehyd der Formel (III) und gegebenenfalls 1.0 bis 3.0 Mol, vorzugsweise 1.0 bis 1.5 Mol an Reaktionshilfsmittel verwendeter Base ein. Die Reaktionsdurchführung, Aufarbeitung und Isolierung der Reaktionsprodukte erfolgt nach bekannten Verfahren (vergleiche hierzu beispielsweise DE 40 12 792; J. Amer. Chem. Soc. 83, 1734 [1961] oder die Herstellungsbeispiele).

Die so erhältlichen 1-Alkoxyhexatrien-2-carbonsäureester der Formel (I) sind in der Regel Gemische aus in Bezug auf die drei Doppelbindungen stereoisomeren Verbindungen, wobei im allgemeinen die E,E,E-Isomeren überwiegen (vergleiche hierzu auch DE 40 12 792). Sie lassen sich durch Anwendung von UV-Bestrahlung oder durch Einwirkung von Doppelbindungs-isomerisierenden Reagenzien, wie beispielsweise Iod in einem geeigneten Losungsmittel, wie beispielsweise Toluol in die E,Z,E-Isomeren umwandeln, welche in ihrer Stereochemie mit dem Naturstoff Strobilurin identisch sind (vergleiche z. B. Liebigs Ann. Chem. 9, 1616 [1984] oder DE 30 25 368).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis; Plasmopara-Arten, wie beispielsweise Plasmopara viticola; Peronospora-Arten, wie beispielsweise Peronospora pisi oder P. brassicae; Erysiphe-Arten, wie beispielsweise Erysiphe graminis; Sphaerotheca-Arten, wie beispielsweise Sphaerotheca fuliginea; Podosphaera-Arten, wie beispielsweise Podosphaera leucotricha; Venturia-Arten, wie beispielsweise Venturia inaequalis; Pyrenophora-Arten, wie beispielsweise Pyrenophora teres oder P. graminea (Konidienform: Drechslera, Syn: Helminthosporium); Cochliobolus-Arten, wie beispielsweise Cochliobolus sativus (Konidienform: Drechslera, Syn: Helminthosporium); Uromyces-Arten, wie beispielsweise Uromyces appendiculatus; Puccinia-Arten, wie beispielsweise Puccinia recondita; Tilletia-Arten, wie beispielsweise Tilletia caries; Ustilago-Arten, wie beispielsweise Ustilago nuda oder Ustilago avenae; Pellicularia-Arten, wie beispielsweise Pellicularia sasakii; Pyricularia-Arten, wie beispielsweise Pyricularia oryzae; Fusarium-Arten, wie beispielsweise Fusarium culmorum; Botrytis-Arten, wie beispielsweise Botrytis cinerea; Septoria-Arten, wie beispielsweise Septoria nodorum; Leptosphaeria-Arten, wie beispielsweise Leptosphaeria nodorum; Cercospora-Arten, wie beispielsweise Cercospora canescens; Alternaria-Arten, wie beispielsweise Alternaria brassicae; Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Getreidekrankheiten, wie beispielsweise gegen den Erreger der Netzfleckenkrankheit der Gerste (Pyrenophora teres) oder gegen den Erreger der Braunfleckenkrankheit an Gerste (Cochliobolus sativus) oder gegen den Erreger der Braunspelzigkeit des Weizens (Septoria nodorum) oder gegen den Erreger des echten Getreidemehltaues (Erysiphe graminis) oder zur Bekämpfung von Krankheiten im Obst- und Gemüseanbau, wie beispielsweise gegen den Erreger der Tomatenbraunfäule (Phytophthora infestans) oder gegen den Erreger des Apfelschorfes (Venturia inaequalis) oder zur Bekämpfung von Reiskrankheiten, wie beispielsweise gegen den Erreger der Reisfleckenkrankheit (Pyricularia olyzae) eingesetzt werden. Dabei besitzen die erfindungsgemäßen Wirkstoffe neben protektiven auch kurative Eigenschaften. Daneben besitzen die erfindungsgemäßen Wirkstoffe eine breite in vitro-Wirksamkeit.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden erflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe wie Cyclohexan oder Paraffine, z.B. Erdölfraktionen, Alkohole wie Butanol oder Glycol sowie deren Ether und Ester, Ketone wie Aceton, Methylethylketon, Methylisobutylketon oder Cyclohexanon, stark polare Lösungsmittel wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase wie Halogenkohlenwasserstoffe sowie Butan, Propan, Stickstoff und Kohlendioxid; als feste Trägerstoffe kommen infrage: z.B. natürliche Gesteinsmehle wie Kaoline, Tonerden, Talkum, Kreide, Quarz, Attapulgit, Montmorillonit oder Diatomeenerde und synthetische Gesteinsmehle wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nichtionogene und anionische Emulgatoren wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

### Herstellungsbeispiele:

### Beispiel 1:

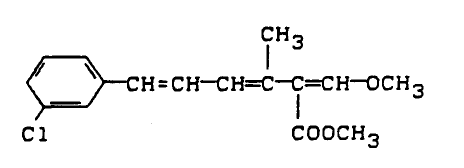

Zu einer Mischung aus 5,6 g (0,04 Mol) 3-Chlorbenzaldehyd und 12,2 g (0,04 Mol) 5-(Diethoxyphosphinyl)-1-methoxy-3-methyl-1,3-pentadien-2-carbonsäuremethylester in 40 ml Tetrahydrofuran gibt man bei -20°C tropfenweise unter Rühren 4,5 g (0,04 Mol) Kalium-tert-Butylat in 40 ml Tetrahydrofuran, läßt die Reaktionsmischung nach beendeter Zugabe auf Raumtemperatur kommen, verdünnt mit Diethylether, wäscht mit Wasser, trocknet über Natriumsulfat und entfernt das Lösungsmittel im Vakuum. Der Rückstand wird durch Chromatographie an Kieselgel (Laufmittel: Diethylether/Petrolether 1:1) gereinigt.

Man erhält 6,3 g (54 % der Theorie) an 1-Methoxy-3-methyl-6-(3-chlorphenyl)-hexatrien-2-carbonsäuremethylester, welches nach GC/MS-Analyse aus folgenden Isomeren besteht:
E,E,E(43,6%) m/e=292,245,233,201,165,155,115,91,75,47,26
E,Z,Z(10,8%) m/e=292,245,233,201,165,155,115,91,75,47,26
E,Z,E(21,1%) m/e=292,245,233,201,165,155,115,91,75,47,26
Z,E,E(11,9%) m/e=292,245,233,201,165,155,115,91,75,47,26

### Isomerisierung des Stereoisomerengemisches:

16 g (0,055 Mol) dieser Mischung werden in 160 ml Toluol mit 0,5 g Iod für 8 Stunden auf Rückflußtemperatur erhitzt, mit weiteren 0,5 g Iod versetzt und weitere 8 Stunden auf Rückflußtemperatur erhitzt. Zur Aufarbeitung wird die Reaktionsmischung an Kieselgel (Laufmittel: Diethylether/Petrolether 1:1) chromatographiert.

Man erhält 16 g Rohgemisch, wovon 5,9 % als E,E,E-Isomeres und 28,2 % als E,Z,E-Isomeres vorliegen. Durch Chromatographie an Kieselgel (Laufmittel : Diethylether/Petrolether 1:1) kann daraus das reine E,Z,E-Isomere isoliert werden.
¹H-NMR (CDCl₃/Tetramethylsilan):
- δ =: 1,98 (3H); 3,74 (3H); 3,86 (3H); 6,269, 6,273 d (1H); 6,44, 6,56 d (1H); 6,6-6,65 dd (1H); 7,44 (1H); 7,12-7,34 m (4H) ppm.

In entsprechender Weise und gemäß den allgemeinen Angaben zur Herstellung erhält man die folgenden 1-Alkoxyhexatrien-2-carbonsäureester der allgemeinen Formel (I):

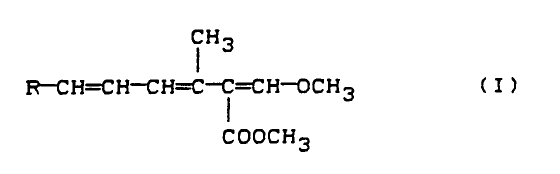

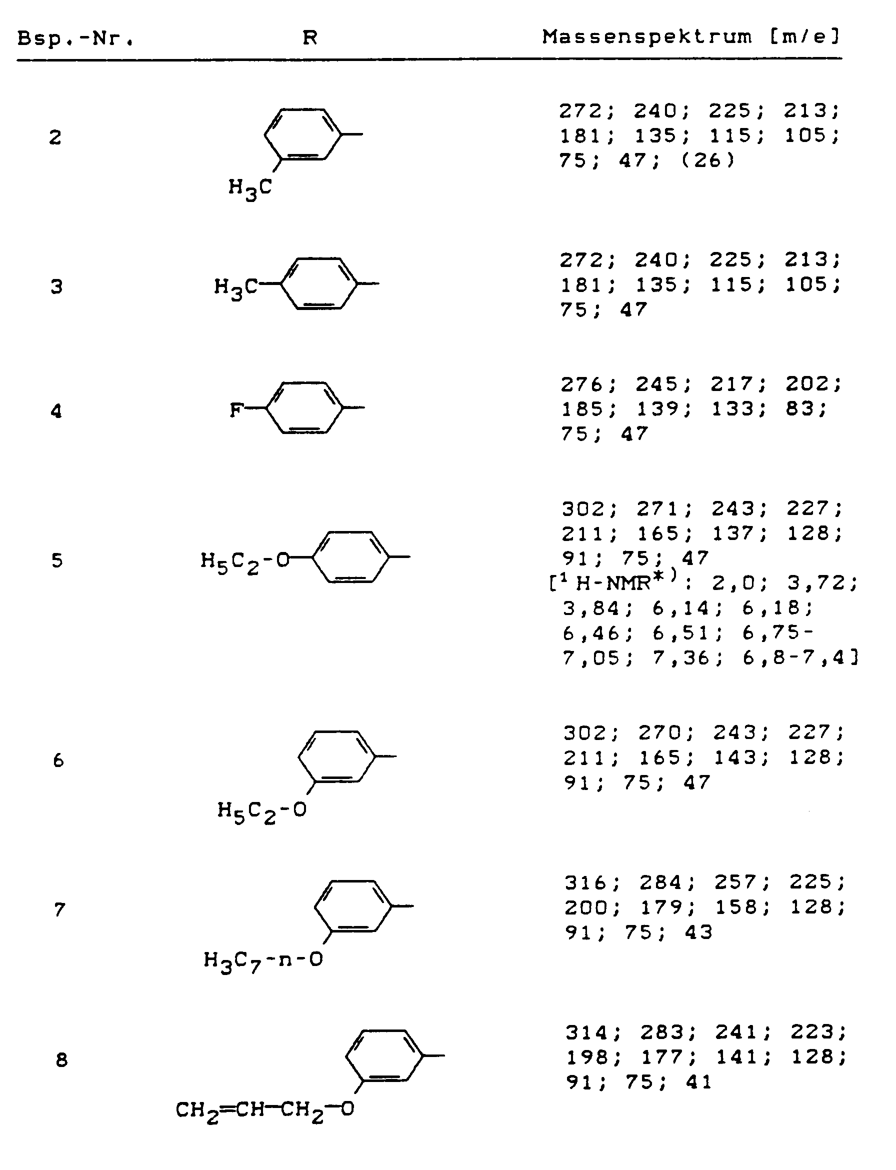

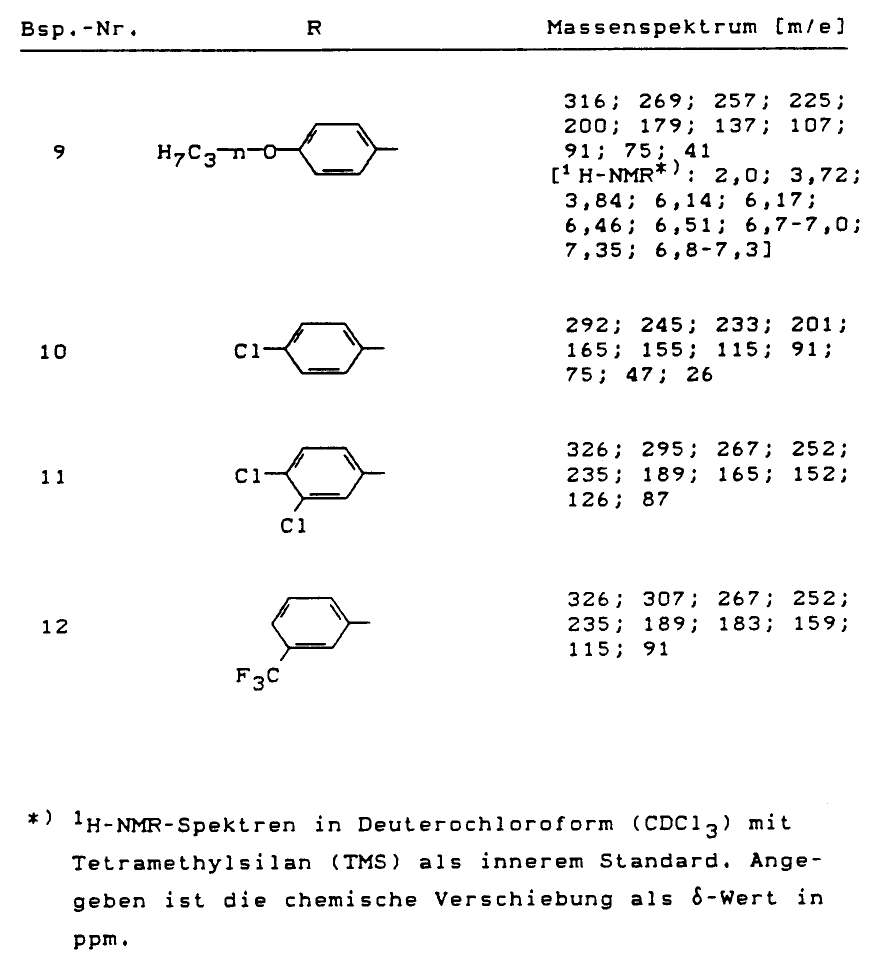

### Anwendungsbeispiele:

In den folgenden Anwendungsbeispielen werden die nachstehend aufgeführten Verbindungen als Vergleichssubstanzen eingesetzt: 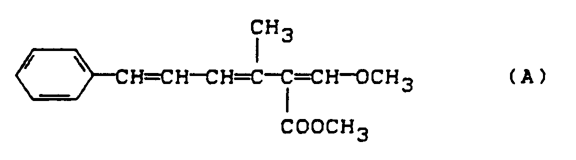
1-Methoxy-3-methyl-6-phenyl-hexatrien-2-carbonsäuremethylester 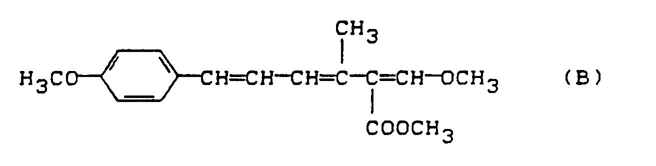
1-Methoxy-3-methyl-6-(4-methoxyphenyl)-hexatrien-2-carbonsäuremethylester

### Beispiel A

### Venturia-Test (Apfel) / protektiv

Lösungsmittel: 4,7 Gewichtsteile Aceton
Emulgator: 0,3 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine mehr als 100 % höhere Wirksamkeit gegenüber dem Stand der Technik zeigen in diesem Test z.B. die Verbindungen gemäß folgender Herstellungsbeispiele: 1, 2, 3, 5, 10 und 11 bei einer Wirkstoffkonzentration von 10 ppm.

### Beispiel B

### Phytophthora-Test (Tomate) / kurativ

Lösungsmittel: 4,7 Gewichtsteile Aceton
Emulgator: 0,3 Gewichtsteile Alky-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20°C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden dann in einer Inkubationskabine bei 20°C und einer relativen Luftfeuchtigkeit von ca. 100 % aufgestellt.

3 Tage nach der Inokulation erfolgt die Auswertung.

Eine um bis zu 90 % höhere Wirksamkeit gegenüber dem Stand der Technik zeigen in diesem Test z.B. die Verbindungen gemäß folgender Herstellungsbeispiele: 4, 5, 7, 9 und 10 bei einer Wirkstoffkonzentration von 50 ppm.

### Beispiel C

### Cochliobolus sativus-Test (Gerste) / protektiv

Lösungsmittel: 100 Gewichtsteile Dimethylformamid
Emulgator: 0,25 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine mehr als 100 % höhere Wirksamkeit gegenüber dem Stand der Technik zeigen bei diesem Test z.B. die Verbindungen gemäß folgender Herstellungsbeispiele: 2 und 4 bei einer Wirkstoffkonzentration von 100 ppm.

### Beispiel D

### Pyrenophora teres-Test (Gerste) / protektiv

Lösungsmittel: 100 Gewichtsteile Dimethylformamid
Emulgator: 0,25 Gewichtsteile Alkyl-Aryl-Polyglykolether
Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Abtrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine mehr als 100 % höhere Wirksamkeit gegenüber dem Stand der Technik zeigt bei diesem Test z.B. die Verbindung gemäß Herstellungsbeispiel 2 bei einer Wirkstoffkonzentration von 100 ppm.

## Patentansprüche

1. 1-Alkoxyhexatrien-2-carbonsäureester der allgemeinen Formel (I), 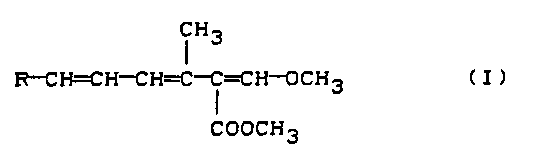 in welcher
R für substituiertes Phenyl steht, wobei als Substituenten infrage kommen:
Halogen, Cyano, Nitro, Alkyl, Alkenyl, Alkinyl, Alkoxy, Alkenyloxy, Alkinyloxy, Alkylthio, Alkylsulfinyl, Alkylsulfonyl, Halogenalkyl, Halogenalkenyl, Halogenalkinyl, Halogenalkoxy, Halogenalkenyloxy, Halogenalkinyloxy, Halogenalkylthio, Halogenalkylsulfinyl, Halogenalkylsulfonyl, Alkoxycarbonyl, Alkoxycarbonylalkyl, Alkoximinoalkyl sowie jeweils gegebenenfalls substituiertes Aralkyl, Aralkyloxy, Aryl oder Aryloxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethyl ester ausgenommen sind.

2. Verbindungen der Formel (I), gemäß Anspruch 1 bei welchen
R für gegebenenfalls einfach oder mehrfach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 8 Kohlenstoffatomen, jeweils geradkettiges oder verzweigtes Alkenyl, Alkinyl, Alkenyloxy oder Alkinyloxy mit jeweils 2 bis 8 Kohlenstoffatomen, jeweils geradkettiges oder verzweigtes Halogenalkyl, Halogenalkoxy oder Halogenalkylthio mit jeweils 1 bis 6 Kohlenstoffatomen und 1 bis 13 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 6 Kohlenstoffatomen und 1 bis 11 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder jeweils geradkettiges oder verzweigtes Alkyl und/oder Alkoxy mit jeweils 1 bis 6 Kohlenstoffatomen substituiertes Benzyl, Benzyloxy, Phenyl oder Phenoxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

3. Verbindungen der Formel (I), gemäß Anspruch 1 bei welchen
R für gegebenenfalls einfach bis fünffach, gleich oder verschieden substituiertes Phenyl steht, wobei als Substituenten infrage kommen:
Halogen, Cyano, Nitro, jeweils geradkettiges oder verzweigtes Alkyl, Alkoxy oder Alkylthio mit jeweils 1 bis 6 Kohlenstoffatomen, jeweils geradkettiges oder verzweigtes Alkenyl, Alkinyl, Alkenyloxy oder Alkinyloxy mit jeweils 2 bis 6 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 4 Kohlenstoffatomen und 1 bis 7 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 4 Kohlenstoffatomen in den einzelnen Alkylteilen sowie gegebenenfalls einfach bis dreifach, gleich oder verschieden durch Halogen und/oder jeweils geradkettiges oder verzweigtes Alkyl und/oder Alkoxy mit jeweils 1 bis 4 Kohlenstoffatomen substituiertes Benzyl, Benzyloxy, Phenyl oder Phenyloxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäure-methylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

4. Verbindungen der Formel (I), gemäß Anspruch 1 bei welchen
R für gegebenenfalls einfach bis dreifach, gleich oder verschieden substituiertes Phenyl steht, wobei als Substituenten infrage kommen:
Fluor, Chlor, Brom, Cyano, Nitro, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, jeweils geradkettiges oder verzweigtes Alkenyl, Alkinyl, Alkenyloxy oder Alkinyloxy mit jeweils 2 bis 4 Kohlenstoffatomen, jeweils geradkettiges oder verzweigtes Halogenalkyl, Halogenalkoxy oder Halogenalkylthio mit jeweils 1 bis 3 Kohlenstoffatomen und 1 bis 7 gleichen oder verschiedenen Halogenatomen, jeweils geradkettiges oder verzweigtes Halogenalkenyl, Halogenalkinyl, Halogenalkenyloxy oder Halogenalkinyloxy mit jeweils 2 bis 4 Kohlenstoffatomen und 1 bis 5 gleichen oder verschiedenen Halogenatomen, jeweils geradkettiges oder verzweigtes Alkoxycarbonyl, Alkoxycarbonylalkyl oder Alkoximinoalkyl mit jeweils 1 bis 3 Kohlenstoffatomen in den einzelnen Alkylteilen sowie gegebenenfalls einfach oder zweifach, gleich oder verschieden durch Fluor, Chlor, Brom, Methyl, Ethyl, n-oder i-Propyl, n-, i-, s-oder t-Butyl, Methoxy, Ethoxy, n-oder i-Propoxy, n-, i-, s-oder t-Butoxy substituiertes Benzyl, Benzyloxy, Phenyl oder Phenoxy,
wobei die Verbindungen 1-Methoxy-3-methyl-6-(4-methoxyphenyl)-hexatrien-2-carbonsäuremethylester, 1-Methoxy-3-methyl-6-(3-methoxyphenyl)-hexatrien-2-carbonsäuremethylester, 1-Methoxy-3-methyl-6-(4-chlor-3-methoxyphenyl)-hexatrien-2-carbonsäuremethylester, 1-Methoxy-3-methyl-6-[2-(3-methyl-but-2-en-1-yl-oxy)-phenyl]-hexatrien-2-carbonsäuremethylester und 1-Methoxy-3-methyl-6-[3-(3-methyl-but-2-en-1-yl-oxy)-phenyl]-hexatrien-2-carbonsäuremethylester ausgenommen sind.

5. Schädlingsbekämpfungsmittel, gekennzeichnet durch einen Gehalt an mindestens einem 1-Alkoxyhexatrien-2-carbonsäureester der Formel (I) nach den Ansprüchen 1 bis 4.

6. Verfahren zur Bekämpfung von Schädlingen, dadurch gekennzeichnet, daß man 1-Alkoxyhexatrien-2-carbonsäureester der Formel (I) nach den Ansprüchen 1 bis 4 auf Schädlinge und/oder ihren Lebensraum einwirken läßt.

7. Vefahren zur Herstellung von 1-Alkoxyhexatrien-2-carbonsäureestern der allgemeinen Formel (I) 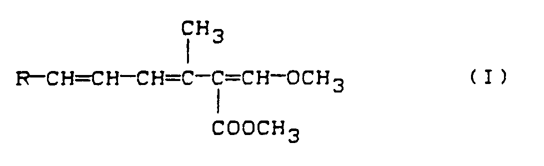 in welcher
R die in Anspruch 1 angegebene Bedeutung hat,
dadurch gekennzeicnet, daß man Methoxymethylencarbonsäureester der Formel (II), 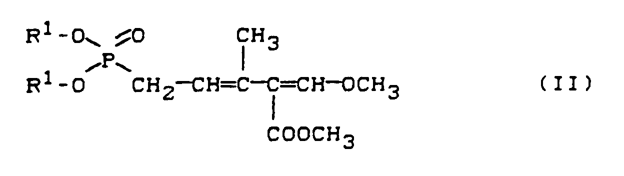 in welcher
R¹ für Alkyl steht,
mit Benzaldehyden der Formel (III),
R-CH=O (III)
in welcher
R die oben angegebene Bedeutung hat,
gegebenenfalls in Gegenwart eines Verdünnungsmittel und gegebenenfalls in Gegenwart eines Reaktionshilfsmittels umsetzt.

8. Verwendung von 1-Alkoxyhexatrien-2-carbonsäureester der Formel (I) nach den Ansprüchen 1 bis 4 zur Bekämpfung von Schädlingen.

9. Verfahren zur Herstellung von Schädlingsbekämpfungsmitteln, dadurch gekennzeichnet, daß man 1-Alkoxyhexatrien-2-carbonsäureester der Formel (I) nach den Ansprüchen 1 bis 4 mit Streckmitteln und/oder oberflächenaktiven Mitteln vermischt.

## Claims

1. 1-Alkoxyhexatriene-2-carboxylates of the general formula (I) 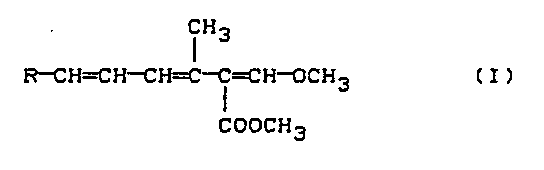 in which
R represents substituted phenyl, suitable substituents being:
halogen, cyano, nitro, alkyl, alkenyl, alkinyl, alkoxy, alkenyloxy, alkinyloxy, alkylthio, alkylsulphinyl, alkylsulphonyl, halogenoalkyl, halogenoalkenyl, halogenoalkinyl, halogenoalkoxy, halogenoalkenyloxy, halogenoalkinyloxy, halogenoalkylthio, halogenoalkylsulphinyl, halogenoalkylsulphonyl, alkoxycarbonyl, alkoxycarbonylalkyl, alkoximinoalkyl and in each case optionally substituted aralkyl, aralkyloxy, aryl or aryloxy,
with the exception of the compounds methyl 1-methoxy-3-methyl-6-(4-methoxyphenyl)-hexatriene-2-carboxylate, methyl 1-methoxy-3-methyl-6-(3-methoxyphenyl)-hexatriene-2-carboxylate, methyl 1-methoxy-3-methyl-6-(4-chloro-3-methoxyphenyl)-hexatriene-2- carboxylate, methyl 1-methoxy-3-methyl-6-[2-(3-methyl-but-2-en-1-yl-oxy)-phenyl]-hexatriene-2-carboxylate and methyl 1-methoxy-3-methyl-6-[3-(3-methyl-but-2-en-1-yl-oxy)-phenyl]-hexatriene-2-carboxylate.

2. Compounds of the formula (I), according to Claim 1, in which
R represents phenyl which is optionally monosubstituted or polysubstituted by identical or different substituents, suitable substituents being:
halogen, cyano, nitro, in each case straight-chain or branched alkyl, alkoxy or alkylthio, each of which has 1 to 8 carbon atoms, in each case straight-chain or branched alkenyl, alkinyl, alkenyloxy or alkinyloxy, each of which has 2 to 8 carbon atoms, in each case straight-chain or branched halogenoalkyl, halogenoalkoxy or halogenoalkylthio, each of which has 1 to 6 carbon atoms and 1 to 13 identical or different halogen atoms, in each case straight-chain or branched halogenoalkenyl, halogenoalkinyl, halogenoalkenyloxy or halogenoalkinyloxy, each of which has 2 to 6 carbon atoms and 1 to 11 identical or different halogen atoms, in each case straight-chain or branched alkoxycarbonyl, alkoxycarbonylalkyl or alkoximinoalkyl, each of which has 1 to 6 carbon atoms in the individual alkyl moieties, and benzyl, benzyloxy, phenyl or phenoxy, each of which is optionally monosubstituted or polysubstituted by identical or different substituents from the series comprising halogen and/or in each case straight-chain or branched alkyl and/or alkoxy, each of which has 1 to 6 carbon atoms,
with the exception of the compounds methyl 1-methoxy-3-methyl-6-(4-methoxyphenyl)-hexatriene-2-carboxylate, methyl 1-methoxy-3-methyl-6-(3-methoxyphenyl)-hexatriene-2-carboxylate, methyl 1-methoxy-3-methyl-6-(4-chloro-3-methoxyphenyl)-hexatriene-2-carboxylate, methyl 1-methoxy-3-methyl-6-[2-(3-methyl-but-2-en-1-yl-oxy)-phenyl]-hexatriene-2-carboxylate and methyl 1-methoxy-3-methyl-6-[3-(3-methyl-but-2-en-1-yl-oxy)-phenyl]-hexatriene-2-carboxylate.

3. Compounds of the formula (I), according to Claim 1, in which
R represents phenyl which is optionally monosubstituted to pentasubstituted by identical or different substituents, suitable substituents being:
halogen, cyano, nitro, in each case straight-chain or branched alkyl, alkoxy or alkylthio, each of which has 1 to 6 carbon atoms, in each case straight-chain or branched alkenyl, alkinyl, alkenyloxy or alkinyloxy, each of which has 2 to 6 carbon atoms, in each case straight-chain or branched halogenoalkyl, halogenoalkoxy or halogenoalkylthio, each of which has 1 to 4 carbon atoms and 1 to 9 identical or different halogen atoms, in each case straight-chain or branched halogenoalkenyl, halogenoalkinyl, halogenoalkenyloxy or halogenoalkinyloxy, each of which has 2 to 4 carbon atoms and 1 to 7 identical or different halogen atoms, in each case straight-chain or branched alkoxycarbonyl, alkoxycarbonylalkyl or alkoximinoalkyl, each of which has 1 to 4 carbon atoms in the individual alkyl moieties, and benzyl, benzyloxy, phenyl or phenyloxy, each of which is optionally monosubstituted to trisubstituted by identical or different substituents from the series comprising halogen and/or in each case straight-chain or branched alkyl and/or alkoxy, each of which has 1 to 4 carbon atoms,
with the exception of the compounds methyl 1-methoxy-3-methyl-6-(4-methoxyphenyl)-hexatriene-2-carboxylate, methyl 1-methoxy-3-methyl-6-(3-methoxyphenyl)-hexatriene-2-carboxylate, methyl 1-methoxy-3-methyl-6-(4-chloro-3-methoxyphenyl)-hexatriene-2-carboxylate, methyl 1-methoxy-3-methyl-6-[2-(3-methyl-but-2-en-1-yl-oxy)-phenyl]-hexatriene-2-carboxylate and methyl 1-methoxy-3-methyl-6-[3-(3-methyl-but-2-en-1-yl-oxy)-phenyl]-hexatriene-2-carboxylate.

4. Compounds of the formula (I), according to Claim 1, in which
R represents phenyl which is optionally monosubstituted to trisubstituted by identical or different substituents, suitable substituents being
fluorine, chlorine, bromine, cyano, nitro, methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, in each case straight-chain or branched alkenyl, alkinyl, alkenyloxy or alkinyloxy, each of which has 2 to 4 carbon atoms, in each case straight-chain or branched halogenoalkyl, halogenoalkoxy or halogenoalkylthio, each of which has 1 to 3 carbon atoms and 1 to 7 identical or different halogen atoms, in each case straight-chain or branched halogenoalkenyl, halogenoalkinyl, halogenoalkenyloxy or halogenoalkinyloxy, each of which has 2 to 4 carbon atoms and 1 to 5 identical or different halogen atoms, in each case straight-chain or branched alkoxycarbonyl, alkoxycarbonylalkyl or alkoximinoalkyl, each of which has 1 to 3 carbon atoms in the individual alkyl moieties, and benzyl, benzyloxy, phenyl or phenoxy, each of which is optionally monosubstituted or disubstituted by identical or different substituents from the series comprising fluorine, chlorine, bromine, methyl, ethyl, n- or i-propyl, n-, i-, s- or t-butyl, methoxy, ethoxy, n- or i-propoxy and n-, i-, s- or t-butoxy,
with the exception of the compounds methyl 1-methoxy-3-methyl-6-(4-methoxyphenyl)-hexatriene-2-carboxylate, methyl 1-methoxy-3-methyl-6-(3-methoxyphenyl)-hexatriene-2-carboxylate, methyl 1-methoxy-3-methyl-6-(4-chloro-3-methoxyphenyl)-hexatriene-2-carboxylate, methyl 1-methoxy-3-methyl-6-[2-(3-methyl-but-2-en-1-yl-oxy)-phenyl]-hexatriene-2-carboxylate and methyl 1-methoxy-3-methyl-6-[3-(3-methyl-but-2-en-1-yl-oxy)-phenyl]-hexatriene-2-carboxylate.

5. Pesticides, characterised in that they contain at least one 1-alkoxyhexatriene-2-carboxylate of the formula (I) according to Claims 1 to 4.

6. Method of combating pests, characterised in that 1-alkoxyhexatriene-2-carboxylates of the formula (I) according to Claims 1 to 4 are allowed to act on pests and/or their habitat.

7. Process for the preparation of 1-alkoxyhexatriene-2-carboxylates of the general formula (I) 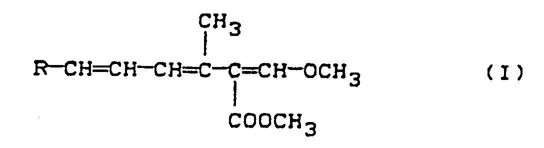 in which
R has the meaning given in Claim 1,
characterised in that methoxymethylenecarboxylates of the formula (II) 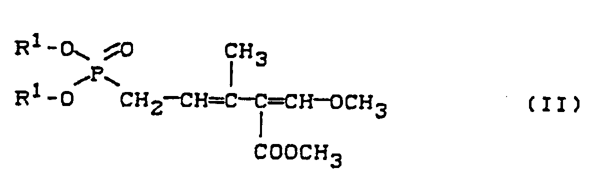 in which
R¹ represents alkyl
are reacted with benzaldehydes of the formula (III)
R-CH=O (III)
in which
R has the abovementioned meaning,
if appropriate in the presence of a diluent and if appropriate in the presence of a reaction auxiliary.

8. Use of 1-alkoxyhexatriene-2-carboxylates of the formula (I) according to Claims 1 to 4 for combating pests.

9. Process for the preparation of pesticides, characterised in that 1-alkoxyhexatriene-2-carboxylates of the formula (I) according to Claims 1 to 4 are mixed with extenders and/or surface-active agents.

## Revendications

1. Esters d'acides 1-alkoxyhexatriène-2-carboxyliques de formule générale (I), 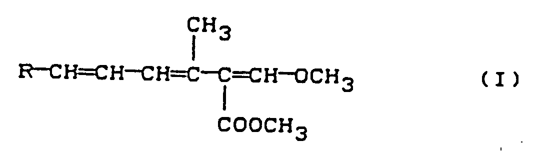 dans laquelle
R représente un groupe phényle substitué, dont les substituants considérés sont :
un halogéne, un radical cyano, nitro, alkyle, alcényle, alcynyle, alkoxy, alcényloxy, alcynyloxy, alkylthio, alkylsulfinyle, alkylsulfonyle, halogénalkyle, halogénalcényle, halogénalcynyle, halogénalkoxy, halogénalcényloxy, halogénalcynyloxy, halogénalkylthio, halogénalkylsulfinyle, halogénalkylsulfonyle, alkoxycarbonyle, alkoxycarbonylalkyle, alkoximinoalkyle ainsi qu'un groupe aralkyle, aralkyloxy, aryle ou aryloxy, dont chacun est éventuellement substitué,
les composés ester méthylique d'acide 1-méthoxy-3-méthyl-6-(4-méthoxyphényl)-hexatriène-2-carboxylique, ester méthylique d'acide 1-méthoxy-3-méthyl-6-(3-méthoxyphényl)-hexatriène-2-carboxylique, ester méthylique d'acide 1-méthoxy-3-méthyl-6-(4-chloro-3-méthoxyphényl)-hexatriène-2-carboxylique, ester méthylique d'acide 1-méthoxy-3-méthyl-6-[2-(3-méthyl-but-2-ène-1-yloxy)-phényl]-hexatriène-2-carboxylique et ester méthylique d'acide 1-méthoxy-3-méthyl-6-[3-(3-méthyl-but-2-ène-1-yloxy)-phényl]-hexatriène-2-carboxylique étant exclus.

2. Composés de formule (I) suivant la revendication 1, dans lesquels
R représente un groupe phényle portant, le cas échéant, un ou plusieurs substituants identiques ou différents, en considérant comme substituants :
un halogène, un radical cyano, nitro, un radical alkyle, alkoxy ou alkylthio linéaire ou ramifié ayant chacun 1 à 8 atomes de carbone, un radical alcényle, alcynyle, alcényloxy ou alcynyloxy linéaire ou ramifié ayant chacun 2 à 8 atomes de carbone, un radical halogénalkyle, halogénalkoxy ou halogénalkylthio linéaire ou ramifié ayant chacun 1 à 6 atomes de carbone et 1 à 13 atomes d'halogènes identiques ou différents, un radical halogénalkyle, halogénalcynyle, halogénalcényloxy ou halogénalcynyloxy linéaire ou ramifié ayant chacun 2 à 6 atomes de carbone et 1 à 11 atomes d'halogènes identiques ou différents, un radical alkoxycarbonyle, alkoxycarbonylalkyle ou alkoximinoalkyle linéaire ou ramifié ayant chacun 1 à 6 atomes de carbone dans les parties alkyle individuelles ainsi que, le cas échéant, un radical benzyle, benzyloxy, phényle ou phénoxy portant chacun, le cas échéant, un ou plusieurs substituants, identiques ou différents, halogéno et/ou alkyle et/ou alkoxy linéaires ou ramifiés ayant chacun 1 à 6 atomes de carbone,
les composés ester méthylique d'acide 1-méthoxy-3-méthyl-6-(4-méthoxyphényl)-hexatriène-2-carboxylique, ester méthylique d'acide 1-méthoxy-3-méthyl-6-(3-méthoxyphényl)-hexatriène-2-carboxylique, ester méthylique d'acide 1-méthoxy-3-méthyl-6-(4-chloro-3-méthoxyphényl)-hexatriène-2-carboxylique, ester méthylique d'acide 1-méthoxy-3-méthyl-6-[2-(3-méthyl-but-2-ène-1-yloxy)-phényl]-hexatriène-2-carboxylique et ester méthylique d'acide 1-méthoxy-3-méthyl-6-[3-(3-méthyl-but-2-ène-1-yloxy)-phényl]-hexatriène-2-carboxylique étant exclus.

3. Composés de formule (I) suivant la revendication 1, dans lesquels
R représente un groupe phényle portant, le cas échéant, un à cinq substituants identiques ou différents, les substituants considérés étant :
un halogène, un radical cyano, nitro, un radical alkyle, alkoxy ou alkylthio linéaire ou ramifié ayant chacun 1 à 6 atomes de carbone, un radical alcényle, alcynyle, alcényloxy ou alcynyloxy linéaire ou ramifié ayant chacun 2 à 6 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un radical halogénalcényle, halogénalcynyle, halogénalcényloxy ou halogénalcynyloxy linéaire ou ramifié ayant chacun 2 à 4 atomes de carbone et 1 à 7 atomes d'halogènes identiques ou différents, un radical alkoxycarbonyle, alkoxycarbonylalkyle ou alkoximinoalkyle linéaire ou ramifié ayant chacun 1 à 4 atomes de carbone dans les parties alkyle individuelles ainsi qu'un radical benzyle, benzyloxy, phényle ou phényloxy portant, le cas échéant, un ou trois substituants, identiques ou différents, halogéno et/ou alkyle et/ou alkoxy linéaires ou ramifiés ayant chacun 1 à 4 atomes de carbone,
les composés ester méthylique d'acide 1-méthoxy-3-méthyl-6-(4-méthoxyphényl)-hexatriène-2-carboxylique, ester méthylique d'acide 1-méthoxy-3-méthyl-6-(3-méthoxyphényl)-hexatriène-2-carboxylique, ester méthylique d'acide 1-méthoxy-3-méthyl-6-(4-chloro-3-méthoxyphényl)-hexatriène-2-carboxylique, ester méthylique d'acide 1-méthoxy-3-méthyl-6-[2-(3-méthyl-but-2-ène-1-yloxy)-phényl]-hexatriène-2-carboxylique et ester méthylique d'acide 1-méthoxy-3-méthyl-6-[3-(3-méthyl-but-2-ène-1-yloxy)-phényl]-hexatriène-2-carboxylique étant exclus.

4. Composés de formule (I) suivant la revendication 1, dans lesquels
R est un groupe phényle portant, le cas échéant, un à trois substituants identiques ou différents, les substituants considérés étant :
le fluor, le chlore, le brome, un radical cyano, nitro, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy, tertio-butoxy, méthylthio, éthylthio, n-propylthio, isopropylthio, un radical alcényle, alcynyle, alcényloxy ou alcynyloxy linéaire ou ramifié ayant chacun 2 à 4 atomes de carbone, un radical halogénalkyle, halogénalkoxy ou halogénalkylthio linéaire ou ramifié ayant chacun 1 à 3 atomes de carbone et 1 à 7 atomes d'halogènes identiques ou différents, un radical halogénalcényle, halogénalcynyle, halogénalcényloxy ou halogénalcynyloxy linéaire ou ramifié ayant chacun 2 à 4 atomes de carbone et 1 à 5 atomes d'halogènes identiques ou différents, un radical alkoxycarbonyle, alkoxycarbonylalkyle ou alkoximinoalkyle linéaire ou ramifié ayant chacun 1 à 3 atomes de carbone dans les parties alkyle individuelles ainsi qu'un radical benzyle, benzyloxy, phényle ou phénoxy portant éventuellement un ou deux substituants, identiques ou différents, fluoro, chloro, bromo, méthyle, éthyle, n-propyle, isopropyle, n-butyle, isobutyle, sec.-butyle, tertio-butyle, méthoxy, éthoxy, n-propoxy, isopropoxy, n-butoxy, isobutoxy, sec.-butoxy ou tertio-butoxy,
les composés ester méthylique d'acide 1-méthoxy-3-méthyl-6-(4-méthoxyphényl)-hexatriène-2-carboxylique, ester méthylique d'acide 1-méthoxy-3-méthyl-6-(3-méthoxyphényl)-hexatriène-2-carboxylique, ester méthylique d'acide 1-méthoxy-3-méthyl-6-(4-chloro-3-méthoxyphényl)-hexatriène-2-carboxylique, ester méthylique d'acide 1-méthoxy-3-méthyl-6-[2-(3-méthyl-but-2-ène-1-yloxy)-phényl]-hexatriène-2-carboxylique et ester méthylique d'acide 1-méthoxy-3-méthyl-6-[3-(3-méthyl-but-2-ène-1-yloxy)-phényl]-hexatriène-2-carboxylique étant exclus.

5. Compositions destinées à la lutte contre des parasites, caractérisées par une teneur en au moins un ester d'acide 1-alkoxyhexatriène-2-carboxylique de formule (I) suivant les revendications 1 à 4.

6. Procédé pour combattre des parasites, caractérisé en ce qu'on fait agir des esters d'acides 1-alkoxyhexatriène-2-carboxyliques de formule (I) suivant les revendications 1 à 4 sur des parasites et/ou sur leur milieu.

7. Procédé de production d'esters d'acides 1-alkoxyhexatriène-2-carboxyliques de formule générale (I) 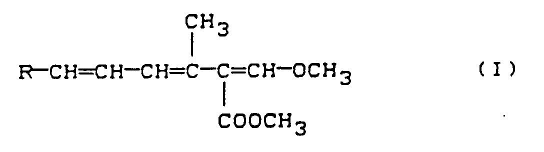 dans laquelle
R a la définition indiquée dans la revendication 1,
caractérisé en ce qu'on fait réagir des esters d'acides méthoxyméthylènecarboxyliques de formule (II) 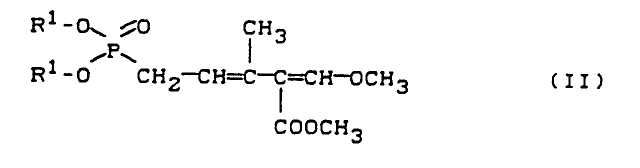 dans laquelle
R¹ est un groupe alkyle
avec des benzaldéhydes de formule (III)
R-CH=O (III)
dans laquelle
R a la définition indiquée ci-dessus,
le cas échéant en présence d'un diluant et en la présence éventuelle d'une substance auxiliaire de réaction.

8. Utilisation d'un ester d'acide 1-alkoxyhexatriène-2-carboxylique de formule (I) suivant les revendications 1 à 4 pour combattre des parasites.

9. Procédé de préparation de compositions pesticides, caractérisé en ce qu'on mélange un ester d'acide alkoxyhexatriène-2-carboxylique de formule (I) suivant les revendications 1 à 4 avec des diluants et/ou des agents tensio-actifs.
